(19) **Europäisches Patentamt**
**European Patent Office**
**Office européen des brevets**

(11) **EP 4 006 137 A1**

(12) # EUROPEAN PATENT APPLICATION
published in accordance with Art. 153(4) EPC

(43) Date of publication:
**01.06.2022 Bulletin 2022/22**

(51) International Patent Classification (IPC):
*C12M 1/32* $^{(2006.01)}$     *C12M 1/12* $^{(2006.01)}$
*C12M 1/26* $^{(2006.01)}$     *C12M 1/34* $^{(2006.01)}$
*G01N 15/00* $^{(2006.01)}$

(21) Application number: **20923237.0**

(22) Date of filing: **06.10.2020**

(86) International application number:
**PCT/KR2020/013587**

(87) International publication number:
**WO 2021/177531 (10.09.2021 Gazette 2021/36)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **06.03.2020 KR 20200028512**

(71) Applicant: **Quantamatrix Inc.**
**Seoul 03082 (KR)**

(72) Inventors:
• **LIM, Tae Geun**
  **Seoul 04421 (KR)**
• **LEE, Gi Yoon**
  **Seoul 08790 (KR)**
• **KIM, Jung Soo**
  **Seoul 08505 (KR)**
• **KIM, Dong Young**
  **Seongnam-si Gyeonggi-do 13597 (KR)**

(74) Representative: **Prüfer & Partner mbB**
**Patentanwälte · Rechtsanwälte**
**Sohnckestraße 12**
**81479 München (DE)**

(54) **RAPID CELL CULTURE-TESTING DEVICE CAPABLE OF PREVENTING SEPARATION OF SOLIDIFIED FLUID**

(57)     Provided is a cell culture test device including a plurality of well units. Each of the well units includes an inlet portion through which a first fluid and a second fluid are introduced and a bottom portion including a first sub-well including a first fluid-accommodating space. The first fluid-accommodating space has an edge groove formed along the outer circumference of the lower end thereof to accommodate a portion of the first fluid or increases in diameter in a direction from the upper end to the lower end thereof.

[FIG. 1]

EP 4 006 137 A1

**Description**

**Technical Field**

The present disclosure relates to a rapid cell culture test device capable of preventing a gelled fluid from being detached, and more specifically to a cell culture test device designed such that a gelled fluid is prevented from being detached from wells of a chip when another fluid is dispensed, facilitating accurate observation.

**Background Art**

Timely prescription of antibiotics for human patients infected with infectious agents greatly affects the survival of the patients. For correct antibiotic prescription, it is first necessary to determine the concentrations of specific antibiotics at which the growth of infection pathogenic strains is inhibited. This determination process is called antibiotic susceptibility testing.

According to a typical antibiotic susceptibility test, whether infectious pathogens are grown after culture in microwells containing various concentrations of a mixture of a culture medium and an antibiotic for about 18 hours is determined by transmittance measurements. A number of products are currently being developed to minimize the time required for culturing infectious pathogens and automate the testing process.

First, miniaturization of culture environments for infectious pathogens is a basic requirement for minimizing the culture time of the infectious pathogens. For such miniaturization and automation, there is a rapidly growing tendency to introduce disposable plastic plates having microscale structures into which microfluids are dispensed and which can be adjusted to desired shapes and sequences.

The present applicant has succeeded in developing an antibiotic susceptibility test system in which a microfluidic plate is introduced such that the growth of bacteria is determined by microscopic imaging. The use of this system is advantageous in that antibiotic susceptibility testing can be completed within a few hours but has problems in that a previously introduced fluid may escape when a subsequent fluid is introduced during movement and fixation of the fluids, resulting in distortion of the antibiotic susceptibility test results. Thus, there is a need for a new solution to these problems.

**Detailed Description of the Invention**

**Problems to be Solved by the Invention**

The present disclosure has been made in an effort to solve the above-described problems and intends to provide a cell culture test device designed such that a gelled fluid is prevented from being detached from wells of a chip when another fluid is dispensed, facilitating accurate observation.

**Means for Solving the Problems**

One aspect of the present disclosure provides a cell culture test device including a plurality of well units, each of which includes an inlet portion through which a first fluid and a second fluid are introduced and a bottom portion including a first sub-well including a first fluid-accommodating space, wherein the first fluid-accommodating space has an edge groove formed along the outer circumference of the lower end thereof to accommodate a portion of the first fluid or increases in diameter in a direction from the upper end to the lower end thereof.

In one embodiment, the first fluid-accommodating space may prevent the first fluid after gelation from being displaced or dislocated when the second fluid is supplied.

In one embodiment, the edge groove may have a thickness of 1 to 2000 $\mu$m and a width of 300 to 2000 $\mu$m.

In one embodiment, the cell culture test device may include bonding means disposed on the rear surface thereof to prevent leakage of the first fluid.

In one embodiment, the bonding means may be an optically transparent film.

In one embodiment, the bonding means may include a pressure control channel through which the first fluid is easily introduced into the first fluid-accommodating space and the edge groove.

In one embodiment, the bottom portion may include a second sub-well in which the second fluid is accommodated and an antibiotic is loaded at a specific location.

In one embodiment, a stepped portion protrudes toward the center of the well unit along the circumference of the inner wall of the well unit such that the well unit has a lower portion whose inner width is smaller than that of an upper portion at the inlet portion side.

In one embodiment, the stepped portion may have an angle of 90° or more with respect to the advancing direction of the second fluid such that the introduced second fluid is prevented from advancing vertically from the bottom portion.

In one embodiment, the stepped portion may have a continuous edge shape along the circumference of the inner wall.

In one embodiment, the height of the stepped portion may be equal to or smaller than the distance from the inner edge of the bottom portion to the center of the bottom portion.

In one embodiment, the inner edge of the bottom portion formed by the bottom portion and the inner wall of the unit may have an angle of 70 to 120° with respect to the bottom portion.

A further aspect of the present disclosure provides a cell analysis method using a cell culture test device including a plurality of well units, each of which includes an inlet portion through which a first fluid and a second fluid are introduced and a bottom portion including a first sub-well including a first fluid-accommodating space and a second sub-well in which the second fluid is accommodated and an antibiotic is loaded, wherein the first fluid-accommodating space has an edge groove formed along the outer circumference of the lower end thereof to accommodate a portion of the first fluid or increases in diameter in a direction from the upper end to the lower end thereof, the method including (a) introducing the first fluid into the first sub-well and gelling the mixture solution to form a solid thin film, (b) introducing the second fluid into the second sub-well to disperse or dissolve the antibiotic until the second fluid reaches the well unit over the second sub-well to come into contact with the solid thin film of the first fluid in the first sub-well such that the antibiotic is diffused into the solid thin film, and (c) observing changes of the biological agent on a single cell basis at the interface between the solid thin film of the first fluid and the second fluid.

In one embodiment, the method may further include (d) observing changes of the biological agent responding to the antibiotic on a single cell basis to determine the minimum inhibitory concentration (MIC) or minimum biofilm eradication concentration (MBEC) of the antibiotic.

**Effects of the Invention**

The rapid cell culture test device of the present disclosure is designed to prevent a gelled fluid from being detached. Due to this design, a solid thin film formed by gelation of a fluid introduced into the rapid cell culture test device can be prevented from being detached as much as possible, allowing for accurate testing compared to conventional antibiotic test devices. The rapid cell culture test device of the present disclosure uses cells immobilized in the solid thin film to observe the behavior of an antibiotic, enabling rapid antibiotic susceptibility testing compared to conventional devices.

In addition, the rapid cell culture test device of the present disclosure can generally observe changes in the growth of single bacteria within several tens of minutes (usually 30 minutes) and changes in the growth of single colonies of bacteria within 4 to 6 hours. Accordingly, the rapid cell culture test device of the present disclosure can more accurately and rapidly determine the effects of a bioactive agent on a biological agent than conventional devices.

Furthermore, the rapid cell culture test device of the present disclosure can observe changes of a biological agent responding to an antibiotic on a single cell colony basis to determine the minimum inhibitory concentration (MIC) or minimum biofilm eradication concentration (MBEC) of the antibiotic, thus being very useful for biofilm assay as well as antibiotic susceptibility testing.

**Brief Description of the Drawings**

FIG. 1 illustrates one embodiment of a cell culture test device.

FIG. 2 illustrates cross-sectional views of a well unit of a cell culture test device according to one embodiment of the present disclosure.

FIG. 3 illustrates a solid thin film formed in a conventional well: (a) a cross-sectional view and a photograph showing the solid thin film in place; and (b) a cross-sectional view and a photograph showing the solid thin film that is displaced by an external force.

FIG. 4 illustrates an antibiotic susceptibility test using a first fluid and a second fluid introduced into a well unit.

FIG. 5 illustrates the movements of molds used to prepare well units: (a) a conventional mold; and (b) a modified mold adapted to the shape of an undercut.

FIG. 6 illustrates the structure of a sacrificial groove formed in a cell culture test device according to one embodiment of the present disclosure.

FIG. 7 illustrates (a) a fluid that is not spread evenly in a portion of an inner wall due to its surface tension and (b) a cross-sectional view thereof.

FIG. 8 illustrates the contact angle for a fluid at an edge where two surfaces of a structure meet each other.

FIG. 9 illustrates the moving distance of a fluid on one surface of a structure and the moving distance of the fluid at an edge where two surfaces of the structure meet each other.

FIG. 10 schematically illustrates the moving behavior of a fluid on a stepped portion.

FIG. 11 schematically illustrates the behavior of a fluid introduced into a well unit.

FIG. 12 schematically illustrates the behavior of a fluid introduced into a well unit with or without a stepped portion.

## Mode for Carrying out the Invention

Embodiments of the present disclosure will now be described in detail with reference to the accompanying drawings.

However, the present disclosure may be embodied in many different forms and should not be construed as limited to the embodiments set forth herein. Rather, these embodiments are provided so that this disclosure will be thorough and complete, and will fully convey the scope of the present disclosure to those skilled in the art. In the drawings, the dimensions, such as widths and thicknesses, of elements may be exaggerated for clarity. The drawings are explained from an observer's point of view. It will be understood that when an element is referred to as being "on" another element, it can be directly on the other element, or one or more intervening elements may also be present therebetween. Those skilled in the art will appreciate that many modifications can be made without departing from the spirit of the disclosure. Throughout the accompanying drawings, the same reference numerals are used to designate substantially the same elements.

On the other hand, terms used herein are to be understood as described below. While such terms as "first" and "second," etc., may be used to describe various elements, such elements must not be limited to the above terms. The above terms are used only to distinguish one element from another. For example, a first element may be referred to as a second element, and likewise a second element may be referred to as a first element.

As used herein, the singular forms "a", "an" and "the" are intended to include the plural forms as well, unless the context clearly indicates otherwise. It will be further understood that the terms "include(s)", "including", "have (has)" and/or "having", when used in this specification, specify the presence of stated features, integers, steps, operations, elements, and/or components, but do not preclude the presence or addition of one or more other features, integers, steps, operations, elements, components, and/or groups thereof. As used herein, the term "and/or" includes any and all combinations of one or more of the associated listed items. Respective steps of the methods described herein may be performed in a different order than that which is explicitly described. In other words, the respective steps may be performed in the same order as described, simultaneously, or in a reverse order.

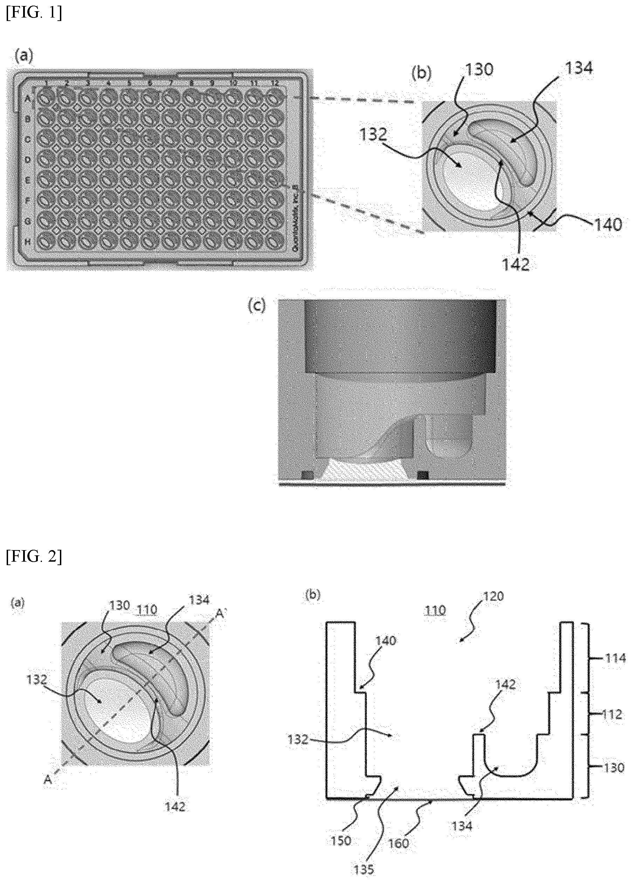

According to one aspect of the present disclosure, there is provided a cell culture test device including a plurality of well units. FIG. 1 illustrates one embodiment of the cell culture test device. In FIG. 1, (a) illustrates the constitution of the cell culture test device, (b) illustrates a partially enlarged view of the cell culture test device, and (c) illustrates a cross-sectional view of one of the well units. The plurality of well units 110 are aligned and may have overall dimensions similar to the wells of a commercial multi-well plate. The centers of the well units 110 may coincide with the centers of the wells of a commercial multi-well plate. Each of the well units 110 includes an inlet portion 120 through which a first fluid and a second fluid are introduced and a bottom portion 130 having a first sub-well 132 and a second sub-sell 134. The first sub-well 132 includes a first fluid-accommodating space and an edge groove 150 is formed along the outer circumference of the lower end of the first fluid-accommodating space to accommodate a portion of the first fluid.

Multi-well plates are standard tools for processing and analyzing a large number of samples in chemical, biochemical, and/or biological assays. Multi-well plates may take various forms, sizes, and shapes. Generally, multi-well plates are designed to have standard sizes and shapes and have standard arrangements of wells. For compatibility with such multi-well plates, the plurality of well units may have standard arrangements of wells, including those found in 96-well plates (12 × 8 array of wells), 384-well plates (24 × 16 array of wells), and 1536-well plates (48 × 32 array of wells). The arrangements of the plurality of well units may be identical or similar to those of various commercially available multi-well plates. The cell culture test device 100 is readily compatible with various conventional biological analysis techniques because its dimensions are similar to those of a commercial multi-well plate. Accordingly, the well units may be arranged in a 1 × 1, 1 × 2, 1 × 4, 2 × 4, 4 × 6, 12 × 8, 24 × 16 or 48 × 32 matrix.

Each of the inlet portions 120 is typically located in the top portion of the corresponding well unit 110 and may be entirely or partially open. The inlet portion 120 may have one or more openings. The first and second fluids may be introduced into the well unit 110 through the inlet portion 120 by pipetting or injection.

The first sub-well 132 of the bottom portion 130 may be recessed in the depth direction and the first fluid-accommodating space 135 of the first sub-well 132 accommodates the first fluid introduced through the inlet portion 120. The second fluid can be directly introduced through the inlet portion 120 but at least a portion of the second fluid introduced through the inlet portion 120 is preferably accommodated in the second sub-well 134 of the bottom portion.

It is preferable to increase the cross-sectional area of the first sub-well 132 as much as possible because changes in the growth of bacteria the first sub-well 132 are observed. To this end, the first sub-well 132 may be designed to have a circular or elliptical shape in cross section and the optional second sub-well 134 may be designed to have an elliptical or crescentic shape in cross section. When each of the first sub-well 132 and the second sub-well 134 has a circular shape in cross section, the sum of the diameters of the first and second sub-wells cannot exceed the diameter of the bottom portion 130. In particular, since a predetermined amount of an antibiotic or the second fluid needs to be introduced into the second sub-well 134, the diameter of the first sub-well 132 is substantially limited to two-thirds of the diameter of the bottom portion 130. In contrast, when the first sub-well 132 has an elliptical shape in cross section, a wider field of view can be secured without the need to change the size of the second sub-well 134. When the second sub-well 134 has an oval or crescentic shape in cross section, the diameter of the first sub-well 132 can be made larger while maintaining the capacity of the second sub-well 134.

The first sub-well 132 and the second sub-well 134 may be physically separated through a partition wall 142. The partition wall 142 serves to separate the first sub-well 132 and the second sub-well 134 from each other in a horizontal direction but does not physically separate the first sub-well 132 and the second sub-well 134 from each other in a vertical direction. Accordingly, the second fluid can cross over the partition wall and be supplied to the first sub-well. Particularly, since an antibiotic is dispersed or dissolved by the second fluid and should come into contact with a solid thin film of the first fluid, it is desirable that the height of the partition wall is similar to those of the side walls of the first sub-well 132 and the second sub-well 134.

Each of the first and second fluids typically includes 70% to 99% by weight, preferably 85% to 99% by weight, most preferably 93% to 99% by weight of water as a dispersion medium or solvent. For example, the first fluid may be a mixture solution of a gelling agent-containing liquid medium and a biological agent. When the content of water in the first fluid is within the range defined above, the water can exhibit optimal performance as a dispersion medium.

The liquid medium of each of the first and second fluids may include water and may be gelled in the presence of a gelling agent. For example, the gelling agent may be agar, agarose, gelatin, alginate, collagen or fibrin. The use of agar or agarose is preferred. For example, agar may be used in an amount of 0.3 to 4% by weight in the liquid medium. The liquid medium usually requires no nutrients. In some examples, however, the liquid medium may include nutrients. The liquid medium may be a solution including a medium stimulating the division of particular cells. If the agar content is less than 0.3% by weight, the liquid medium may not be gelled. Meanwhile, if the agar content exceeds 4% by weight, the liquid medium may be excessively gelled, making it difficult to diffuse the biological agent.

Examples of suitable biological agents include viruses, bacteria, fungi, algae, protozoa, parasitic pathogens, human and mammalian cells, and biofilms. The biological agent may be a mixture of an infectious biological agent (*e.g.*, a bacterial, viral or fungal strain) and blood cells. For example, the biological agent may be blood collected from a human infected with bacteria. In this case, a medium stimulating the division of only the bacterial cells can be used to distinguish the bacterial cells from the blood cells based on their different sizes. The biological agent may grow in a liquid or solid medium and the growth of the biological agent may be affected by the kind and concentration of a foreign bioactive agent. The density of the biological agent in the mixture solution is from $10^2$ to $10^{10}$ cells/ml, preferably from $10^4$ to $10^9$ cells/ml, more preferably from $10^5$ to $10^8$ cells/ml. If the density of the biological agent is below the lower limit, it may be difficult to perceive the location of the biological agent. Meanwhile, if the density of the biological agent exceeds the upper limit, it may be difficult to perceive the individual state of the biological agent.

The bottom portion 130 or the first sub-well 132 may include an antibiotic in a solid form. In this case, the antibiotic may be in the form of a dried solid. The solvent is capable of dissolving the dried antibiotic and may be selected from water, cell culture media, dimethylformamide (DMF), and dimethyl sulfoxide (DMSO). The solvent may be introduced by pipetting such that the antibiotic in a solid form is completely dissolved and the resulting solution has a uniform concentration. Any drying process that does not deteriorate the efficacy of the antibiotic may be used without limitation to dry the antibiotic. The antibiotic is preferably dried by heat drying, freeze drying or vacuum drying, most preferably heat drying. The antibiotic thus dried is introduced into the bottom portion or the first sub-well. Alternatively, the antibiotic solution may be introduced into the bottom portion or the first sub-well and dried by the drying process mentioned above.

In the case where the cell culture test device includes the second sub-wells 134, it is preferable that a dried antibiotic is present in the second sub-wells. In this case, the second fluid is preferably introduced into the second sub-wells to dissolve the antibiotic present in the second sub-wells and fills the bottom portions 130 and lower portions 112 of the well units.

The second fluid may be a solution containing an antibiotic or a solvent dissolving an antibiotic. As a result, the second fluid containing an antibiotic may be loaded in the second sub-wells 134. The volume of the second sub-well 134 may vary depending on a desired scale of the system but may be determined between 1 $\mu$l and 50 $\mu$l, which is an achievable range. If the volume of the second sub-well is less than 1 $\mu$l, the reduced size of the well makes pipetting and drying difficult. Meanwhile, if the volume of the second sub-well exceeds 50 $\mu$l, large amounts of a sample and reagents are required, resulting in a reduction in efficiency.

FIG. 2 illustrates one of the well units of the cell culture test device according to the present disclosure. In FIG. 2, (a) is a top view of the well unit 110 of the cell culture test device and (b) is a cross-sectional view taken along line A-A' of (a). The first sub-well 132 may be in the form of a well that has a sufficient space to accommodate the first fluid. The volume of the first sub-well 132 is not particularly limited and may be large enough to observe responses for a long time after introduction of the first fluid. The volume of the first sub-well 132 may be, for example, 1 µl to 50 µl. If the volume of the first sub-well is less than 1 µl, the reduced size of the well is not suitable for observation. Meanwhile, if the volume of the first sub-well exceeds 50 µl, large amounts of a sample and reagents are required, resulting in a reduction in efficiency.

The greatest features of the present disclosure are that the first fluid-accommodating space 135 of each of the first sub-wells 132 accommodates the first fluid and the edge groove 150 formed along the outer circumference of the lower end of the first fluid-accommodating space accommodates a portion of the first fluid or the first fluid-accommodating space increases in diameter in a direction from the upper end to the lower end thereof.

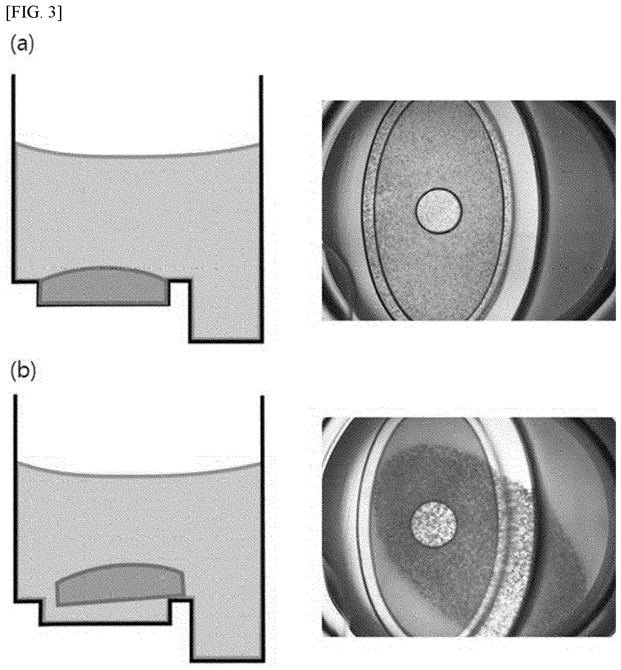

The edge groove 150 is located in a hidden portion as viewed from the top of the sub-well, as illustrated in FIG. 2, but it can accommodate a portion of the first fluid supplied to the first sub-well 132. A conventional disposable plastic chip (or a multi-well plate) is typically manufactured by injection molding and includes wells designed so as not to have an undercut shape in which the upper end is wider than the lower end. A single-body product having no undercut shape is easy to remove from a mold after injection molding and undergoes minimal damage during removal from the mold. However, the single-body product has a disadvantage in that when a first fluid is supplied to form a solid thin film, as in the present disclosure, the solid thin film tends to be displaced or dislocated when an external force is applied because of its characteristics. The displacement or dislocation of the solid thin film may act as a factor impeding the reliability of a system designed to inspect the locations of targets. In particular, this displacement or dislocation may lead to acquisition of an incorrect microscopy image or even failure to acquire a microscopy image. As illustrated in (a) of Fig. 3, the location of the solid thin film in place enables easy observation. However, as illustrated in (b) of Fig. 3, the solid thin film may be displaced or dislocated by the dispensed second fluid, making it not easy to observe experimental results.

For a conventional multi-well plate, displacement or dislocation is prevented by chemically treating the bottom surface or forming protrusions on the bottom and wall surfaces. This chemically treating the bottom surface or forming protrusions can make a frictional force. However, the chemical treatment may affect target cells and its effects are known to be insignificant. The formation of protrusions may distort the observation of targets by microscopy, resulting in an incorrect microscopy image.

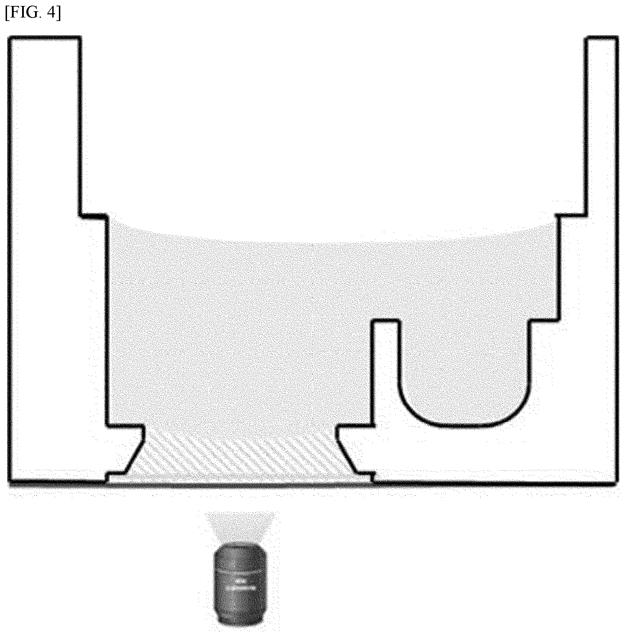

In the present disclosure, a portion of the first fluid supplied to the first fluid-accommodating space of the first sub-well 132 is accommodated in the edge groove 150, as illustrated in FIG. 4. The supplied first fluid is gelled to form a solid thin film. At this time, the first fluid accommodated in the edge groove 150 may also be gelled. The subsequent supply of the second fluid acts as an external force to exert a pressure against the solid thin film. However, since the solid thin film is secured by the gelled product located in the edge groove 150, its displacement and dislocation can be minimized. To this end, the edge groove 150 may be formed to have dimensions of 1 to 2000 µm in thickness and 300 to 2000 µm in width. Within these numerical ranges, the displacement of the solid thin film is effectively prevented by the first fluid accommodated in the edge groove 150. In contrast, if the dimensions of the edge groove 150 are less than the respective lower limits, the effect of preventing the displacement and dislocation of the solid thin film is insignificant. Meanwhile, if the dimensions of the edge groove 150 exceed the respective upper limits, the amount of the first fluid flowing into in the edge groove 150 increases, which is inefficient because an increased amount of a sample is necessary for inspection.

The first fluid-accommodating space increases in diameter in a direction from the upper end to the lower end thereof. The first fluid-accommodating space accommodates the first fluid supplied to the first sub-well 132. The first fluid-accommodating space whose diameter increases from the upper end to the lower end thereof, as illustrated in FIGS. 2 and 3, serves to prevent the solid thin film of the first fluid from being displaced or dislocated upward. The first fluid-accommodating space 135 may have a circular or elliptical shape in cross section, similarly to the first sub-well 132. In particular, the first fluid-accommodating space 135 has an elliptical cross-sectional shape whose major or minor axis or both of them are set such that the lower end is larger than the upper end. This shape prevents the solid thin film from being detached. The wall surface of the first fluid-accommodating space may have an inverted slope at an angle of 1 to 80° with respect to the vertical line. If the wall surface of the first fluid-accommodating space has an inverted slope at an angle of less than 1°, the first fluid-accommodating space has no effect on preventing the solid thin film from being detached. Meanwhile, if the wall surface of the first fluid-accommodating space has an inverted slope at an angle exceeding 80°, the first fluid-accommodating space is made low in height, making the thickness of the solid thin film unsuitable for observation.

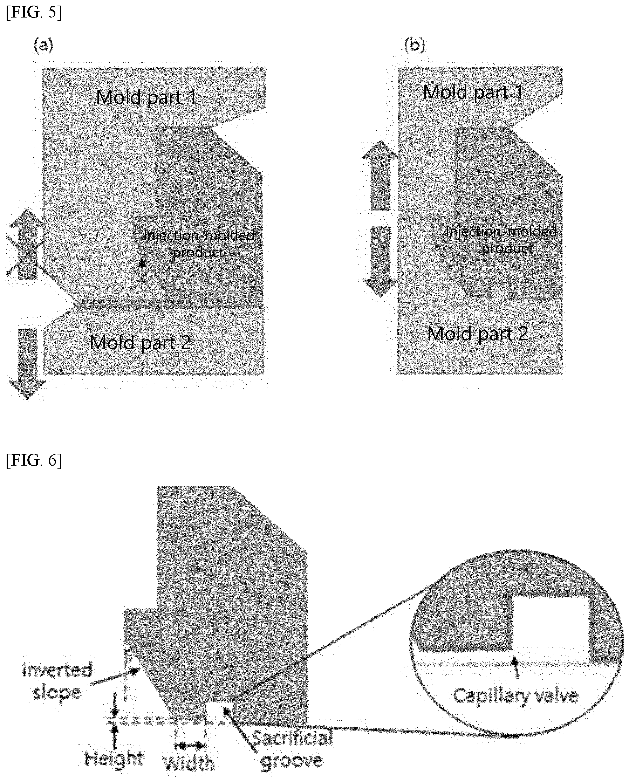

The diameters of the lower ends of the first fluid-accommodating space 135 and the edge groove 150 are larger than that of the upper end of the first fluid-accommodating space to form an undercut shape. Because the undercut shape is difficult to form by simple injection molding, it can be formed by injection molding using a lower mold part for manufacturing well plates in which the shapes of the first fluid-accommodating space 135 and the edge groove 150 are formed. When a lower mold part prepared by a conventional method is used, the mold is difficult to separate due to the undercut shape in which the lower ends of the first fluid-accommodating space and the edge groove are larger than the upper end of the first fluid-accommodating space ((a) of FIG. 5). In contrast, the use of a lower mold part in which the first fluid-accommodating space and the edge groove are formed facilitates the separation of the mold because there is no undercut, as illustrated in (b) of FIG. 5.

Since the lower ends of the first fluid-accommodating space 135 and the edge groove 150 are exposed to the outside, the first fluid cannot be accommodated in the first fluid-accommodating space 135 and the edge groove 150. Thus, bonding means 160 is bonded to the rear surface of the cell culture test device to prevent leakage of the first fluid and accommodate the first fluid in the first fluid-accommodating space 135 and the edge groove 150. Here, the bonding means 160 may be designed to have the same size as the rear surface of the well plate and one bonding means may be bonded to the rear surfaces of the well units. Alternatively, different bonding means may be separately bonded to the rear surfaces of the well units. In this case, each of the bonding means 160 may be adapted to the size of the corresponding well unit. It is preferable to use an optically transparent film as the bonding means because the solid thin film formed in the lower portion of the first sub-well 132 is observed through the rear surface of the well plate.

The bonding means may include a pressure control channel. The pressure control channel is used as a passage through which air present in the first fluid-accommodating space and the edge groove is released to the outside when the first fluid is introduced. The pressure control channel communicates with the first fluid-accommodating space or the edge groove and is exposed to the atmosphere. This configuration allows air present in the first fluid-accommodating space and the edge groove to be released to the outside when the first fluid-accommodating space and the edge groove are filled with the first fluid. Particularly, it is more preferable that the pressure control channel communicates with the edge groove because the first fluid is first introduced into the first fluid-accommodating space and then introduced into the edge groove by capillary action.

It is preferable that the pressure control channel is connected to the first fluid-accommodating space or the edge groove through a capillary valve. When the pressure control channel is in direct communication with the first fluid-accommodating space or the edge groove, a portion of the first fluid may be introduced into the pressure control channel. Particularly, the thickness of the solid thin film formed by gelation of the first fluid may vary depending on the amount of the fluid due to the structure of the first fluid-accommodating space. The varying thickness of the solid thin film may affect the analysis. Therefore, it is preferable that a capillary valve is formed between the pressure control channel and the first fluid-accommodating space or the edge groove to always maintain the same amount of the first fluid in the first fluid-accommodating space. The capillary valve prevents the first fluid from flowing into the pressure control channel while releasing air from the pressure control channel and the first fluid-accommodating space or the edge groove to the outside. That is, the thickness and width of the capillary valve are controlled to maintain capillary action. The capillary valve typically has a thickness of 1 $\mu$m to 500 $\mu$m and a width of 200 $\mu$m to 2 mm. With these dimensions, the capillary valve can prevent the first fluid from flowing into the pressure control channel and release only air to the outside. However, if the dimensions of the capillary valve are less than the respective lower limits, air may not be readily released. Meanwhile, if the dimensions of the capillary valve exceed the respective upper limits, the first fluid may flow into the pressure control channel.

FIG. 4 illustrates an antibiotic susceptibility test using the first fluid and the second fluid introduced into the well unit.

The first fluid may be a mixture solution of a gelling agent-containing liquid medium and a biological agent. The first fluid is introduced into the first fluid-accommodating space located in the lower portion of the first sub-well 132 and gelled. As a result of the gelation, the biological agent (e.g., a bacterial strain) is immobilized in the gel matrix. Thereafter, the second fluid is introduced through the inlet portion 120 to dissolve an antibiotic. As the volume of the second fluid gradually increases, the second fluid fills the lower portion of the well unit 110 to form an interface with the gel matrix filled in the first sub-well 132. As a result, the antibiotic is diffused into the gel matrix through the interface and changes in the growth of bacteria immobilized in the gel matrix can be observed with a microscope to determine the susceptibility of the bacteria to the antibiotic.

The supplied first fluid forms a solid thin film after gelation. Here, a portion of the first fluid is accommodated and gelled in the edge groove. The first fluid-accommodating space together with the gelled first fluid can act as a holder to prevent the solid thin film from being detached when an external force is applied thereto.

More specifically, the first fluid entering the first sub-well is supplied to the first fluid-accommodating space located at the lower end of the first sub-well and the edge groove formed on the outer circumference of the first fluid-accommodating space. The first fluid-accommodating space has an upper end and a lower end having a larger diameter than the upper end, as described above. The structure of the first fluid-accommodating space prevents a solid thin film formed by gelation of the first fluid from being detached toward the inlet portion. The edge groove is filled with the first fluid by capillary action and surface tension.

Thereafter, the first fluid supplied to the first sub-well is gelled to form a solid thin film. Here, the first fluid introduced into and gelled in the edge groove serves to fix the solid thin film. For a conventional well plate having an undercut shape, the flow direction of the second fluid supplied coincides with the direction of the interface between the solid thin film and the wall surface of the well unit, with the result that the second fluid may cause displacement and dislocation of the solid thin film and may flow to the lower portion of the solid thin film, making it difficult to acquire an accurate image. In contrast, for the rapid cell culture test device of the present disclosure, although the second fluid flows into the interface between the solid thin film and the wall surface of the well unit when the first fluid is introduced, the solid thin film is prevented from being displaced and dislocated by the first fluid gelled in the edge groove. In addition, the gelled first fluid prevents the second fluid from flowing along the outer surface thereof to block the penetration of the second fluid into the bottom of the solid thin film, contributing to the minimization of image distortion resulting from the entering second fluid.

For microscopic observation, at least a portion of the bottom face of the well unit may be made of an optically transparent material such that the target present in the first fluid is observable through the bottom face where the first sub-well 132 is located. In the present disclosure, the bonding means constituting the bottom face of the first sub-well is made of a transparent material for easy optical observation. The transparent material may be a polymer resin such as polystyrene, polyethylene, polypropylene, polymethacrylate or polycarbonate. For the purposes of ease of bonding and volume reduction after bonding, the bonding means may be an optically transparent film such as a polyethylene, polyvinyl chloride or polystyrene film. The well units of the cell culture test device may be prepared by injection molding of a polymer resin.

When the bottom surface is formed using the bonding means, an adhesive may be used to attach the bonding means to the well plate. At this time, the adhesive may affect experimental results upon contact with the first fluid. The contact of the adhesive with the first fluid is preferably minimized by forming a sacrificial groove on the outer circumference of the edge groove 150. The sacrificial groove is designed to be higher than the edge groove 150 to form a capillary valve, which makes it difficult for the first fluid filled in the edge groove to escape therethrough. Therefore, the sacrificial groove blocks the adhesive from coming into direct contact with the first fluid and ensures stable loading of the first fluid (see FIG. 6).

A stepped portion 140 protrudes toward the center of the well unit 110 along the circumference of the inner wall of the well unit 110. As can be seen from the cross-sectional shape of the well unit 110, the presence of the stepped portion 140 allows the well unit 110 to have a lower portion 112 whose inner width is smaller than that of an upper portion 114 at the inlet portion side. The stepped portion 140 may have a continuous edge shape along the circumference of the inner wall. Due to the shape of the stepped portion 140, the behaviors of the fluids in the well unit 110 are controlled such that the fluids can be evenly spread throughout the entire well unit 110. The function of the stepped portion 140 will be specifically described below.

Factors that have the greatest influence on the behavior of a fluid introduced into a fixed structure are the surface tension acting on the fluid and the structure surface and the gravitational force acting on the fluid as well as the inertial force. However, as the volume of a fluid decreases, the surface tension, which is affected by the surface area of the fluid rather than by the mass of the fluid, becomes a dominant force and the gravitational force has a relatively insignificant effect. Accordingly, the gravitational force has little or no effect on water and the surface tension mainly affects the behavior of the fluid in the volume range of several to several hundreds of $\mu$l, which is mainly employed in the cell culture test device of the present disclosure.

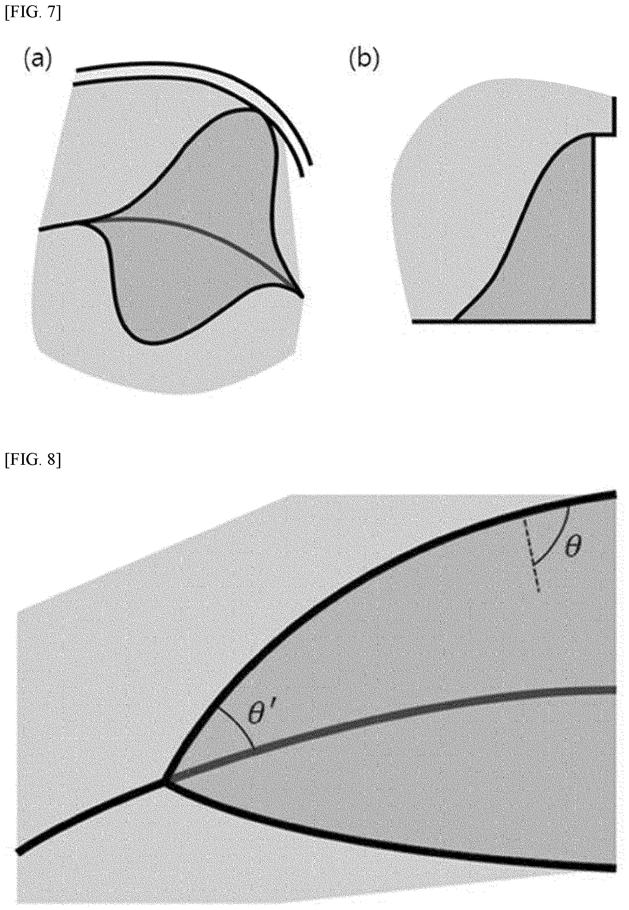

FIG. 7 illustrates (a) the fluid that is not spread evenly in a portion of the inner wall due to its surface tension and (b) a cross-sectional view thereof.

First, in the case where the second fluid is trapped in a portion of the structure and is not spread evenly, which is contradictory to its purpose, the fluid has a length greater than the width thereof and is concentrated toward the bottom rather than the top under the influence of gravity. Due to its low contact angle, the fluid has a sharp shape at the interface with the structure ((a) of FIG. 7) and is convex in cross section because it is distributed over a small area for its volume ((b) of FIG. 7).

In order for the fluid introduced into this environment to fill up the structure, as described above, it is first desirable that the contact angle of the structure surface for the fluid is maintained within 90° and it is also preferable that the surface tension of the fluid is lower than or equal to that of water, which is the strongest except those of some fluids. More specifically, it is preferable that the contact angle for the fluid is 10° to 90°. It is also preferable that the fluid has a density of 0.1 g/mL to 10 g/mL, a viscosity of 0.1 mPa/s to 10 mPa/s, and a volume of 10 $\mu$l to 200 $\mu$l. Within the density and viscosity ranges, the fluid can be completely spread in the structure. Meanwhile, if the density and viscosity of the fluid exceed the respective ranges defined above, the fluid may not be spread evenly in the structure. Here, a description will be given based on the assumption that the fluid has surface tension, density, and viscosity values similar to water (72 dyne/cm, 1 g/mL, and 1 mPa/s, respectively), a contact angle of 50°, and a volume of approximately 100 $\mu$l.

When the contact angle is about 50°, the fluid is basically spread only slightly and aggregates in a rounded shape on the surface of the well unit structure but can move farther at the inner edge of the bottom portion where two surfaces of the structure meet each other. As illustrated in FIG. 8, the contact angle between the fluid and the surface of the structure is lowered at the edge where two surfaces of the structure meet each other, allowing the fluid to spread over a longer distance.

More specifically, when the contact angle ($\theta$) between the fluid and one surface of the structure satisfies the Concus-Finn condition (for example, the contact angle ($\theta$) between the fluid and one surface of the structure is 45° or less when two surfaces of the structure form an angle of 90°), the fluid will theoretically move an infinite distance along the edge. However, if the contact angle ($\theta$) between the fluid and one surface of the structure is larger than the Concus-Finn condition and is smaller than 90°, the contact angle ($\theta$) formed by the fluid and two surfaces of the structure at the edge can be calculated by Equation 1:

[Equation 1]

$$\theta' = \cos^{-1}\left(\frac{\cos\theta}{\sin\theta}\right)\left(\frac{\pi}{4} < \theta < \frac{\pi}{2}\right)$$

where the contact angle ($\theta$) is always smaller than the contact angle ($\theta$), which can be explained by Equation 2:

[Equation 2]

$$\cos\theta' = \frac{\cos\theta}{\sin\theta} \implies \cos\theta' > \cos\theta \implies \theta' < \theta$$

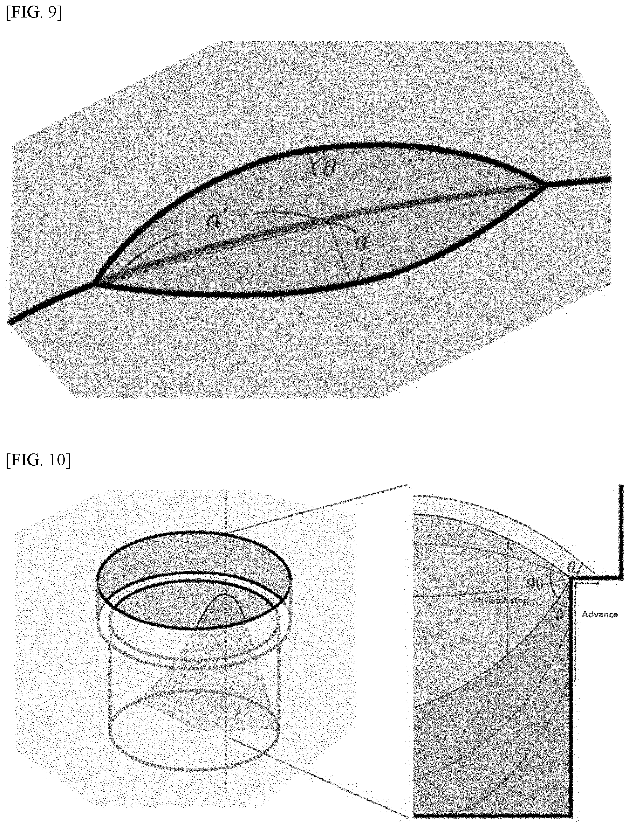

The moving distance ($a$) of the fluid on one surface of the structure and the moving distance ($a'$) of the fluid at the edge where the two surfaces meet each other can be expressed by Equation 3 (see FIG. 9):

[Equation 3]

$$\frac{a'}{a} = \sqrt{\frac{1 + \frac{\cos\theta}{\sin\theta}}{1 - \frac{\cos\theta}{\sin\theta}}} \quad \left(0 < \frac{\cos\theta}{\sin\theta} < 1\right)\left(\frac{\pi}{4} < \theta < \frac{\pi}{2}\right)$$

It is therefore preferable that the inner edge of the bottom portion formed by the bottom portion and the inner wall of the unit has an angle of 70 to 120° with respect to the bottom portion. If the angle of the inner edge is less than 70°, the size of the inlet portion is reduced, making it difficult to introduce the fluid, and the size of the well unit is reduced, making it difficult to conduct the test. Meanwhile, if the angle of the inner edge exceeds 120°, the moving distance of the fluid along the edge is shortened, making it difficult to introduce the fluid.

In contrast, when the fluid meets a step perpendicular to its advancing direction, its advance is stopped until its volume increases such that the contact angle increases by 90° (see FIG. 10). Based on the above condition, the present disclosure uses a step between the edged structure at the bottom face of the target space and the upper portion of the target space to achieve a uniform distribution of the fluid (FIG. 10).

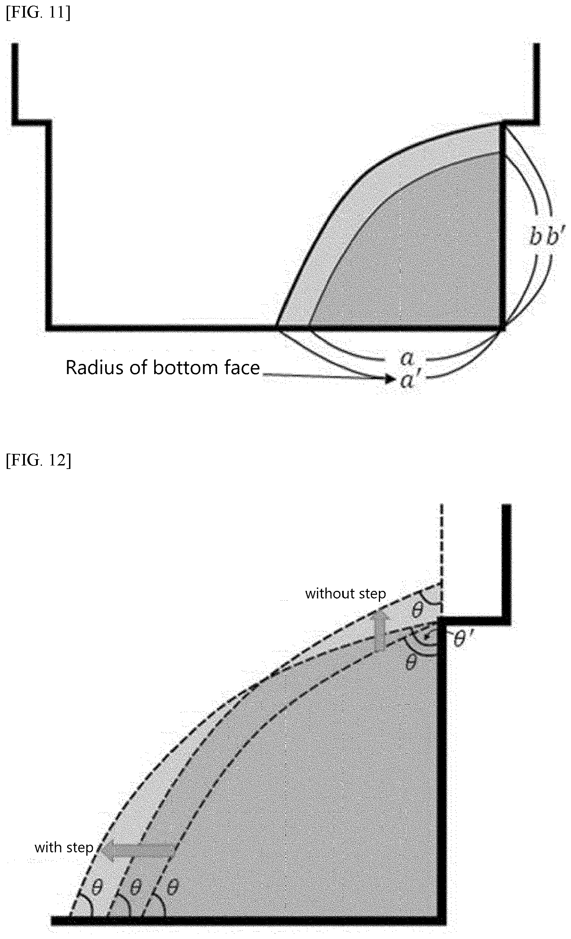

More specifically, the fluid introduced into the well unit 110 climbs up the wall due to its surface tension. Here, the climbing height of the fluid is determined by a balance between the surface tension and the gravitational force of the fluid (FIG. 9). The lengths of the region covered by the fluid on the wall and the bottom face ($a$ and $b$ in FIG. 11, respectively) increase with increasing volume of the fluid and are affected by the gravitational force. Therefore, the lengths ($a, b$) of the region covered by the fluid on the wall and the bottom face satisfy the relationship $a \geq b$.

The stepped portion plays a role in impeding the vertical advance of the fluid to reduce the surface area covered by the fluid. Another role of the stepped portion is to increase the effect of the gravitational force, with the result that a force is applied in a direction horizontal to the fluid and a further advance of the fluid is made. As illustrated in FIG. 12, the fluid may be concentrated in the horizontal direction when a step is provided, unlike when no step is provided. Since the advance of the fluid in the presence of a step is limited to a direction opposite to the direction toward the step, the above design is preferably applied when the height ($b'$) of the stepped portion is smaller than the radius ($a'$) of the bottom face. That is, $a'$ and $b'$ satisfy the relation of: $0 < b' < a'$.

The height at which the fluid has a uniform surface due to the gravitational force is calculated by Equation 4:

[Equation 4]

$$h = \sqrt{2\frac{\gamma}{\rho g}}$$

In the well unit whose height is equal to or greater than $h$, the fluid can be sufficiently spread even without a step. It is thus preferable that the height ($b'$) of the stepped portion is smaller than the height ($h$) calculated by Equation 4 (that is, $b' < h$). The height (h) may vary depending on the kind of the fluid. Since the height (h) is ~4 mm for water, the height ($b'$) of the stepped portion is preferably limited to less than 4 mm in the present disclosure using the first fluid whose height ($h$) is similar to or lower than that of water.

The height of the stepped portion may vary depending on the volume of the second fluid. For example, assuming that the volume of the second fluid is 100 µl and the diameter of the bottom face of each well of a 96-well plate is approximately 8 mm, the height of the stepped portion is at least 0.5 mm. Assuming that the volume of the second fluid is 10 µl and the diameter of the bottom face of each well of a 384-well plate is 4 mm, the height of the stepped portion is 0.2 mm. It is therefore preferable that the height ($b'$) of the stepped portion is 0.2 mm or greater.

That is, the height of the stepped portion is preferably 0.2 to 4 mm. If the height of the stepped portion is less than 0.2 mm, the second fluid reaches the top of the stepped portion, eliminating the advantage of using the stepped portion. Meanwhile, if the height of the stepped portion exceeds 4 mm, the fluid can be sufficiently spread even without the step, which also eliminates the advantage of using the stepped portion.

In the cell culture test device of the present disclosure, the presence of the stepped portion prevents the fluid from rising up above a predetermined height, and at the same time, the presence of the inner edge of the bottom portion promotes the movement of the fluid, with the result that the fluid can be dispensed evenly and quickly.

The thickness and width of the solid thin film are determined depending on the width of the first sub-well and the amount of the first fluid introduced. As used herein, the term "thin film" refers to a thin layer that has a thickness sufficient to immobilize the biological agent and to observe single cells. The thickness of the thin film is typically in the range of 1 µm to 5 mm, 1 µm to 3 mm, 1 µm to 2 mm, 1 µm to 1.5 mm, 1 µm to 1 mm, 1 µm to 800 µm, 1 µm to 500 µm, 1 µm to 100 µm, 10 µm to 3 mm, 100 µm to 500 µm, 10 µm to 1 mm, 100 µm to 1 mm, 200 µm to 1 mm or 500 µm to 1 mm, but is not particularly limited to this range. The thickness of the solid thin film may correspond to the size of a side of the solid thin film in a direction perpendicular to a side of the solid thin film to be observed. Within the thickness range of the solid thin film defined above, the biological agent immobilized in the solid thin film can be observed on a single cell basis.

As described above, the use of the cell culture test device can minimize displacement or dislocation of the solid thin film formed by gelation of the first fluid introduced into the edge groove when the second fluid is dispensed and enables accurate imaging of changes in the growth of a target biological agent during antibiotic susceptibility testing using an optical microscope.

As illustrated in FIG. 1, the first fluid-accommodating space forms a step relative to the second sub-well and is physically separated from the second sub-well through a partition wall. However, the formation of the step may lead to bubble formation at the edge of the bottom surface when the second fluid is introduced into the second sub-well and supplied to the top of the first sub-well. The bubble formation can be prevented by making the wall surface of the first sub-well curved at a predetermined angle of inclination. Particularly, when the wall surface of the first sub-well adjacent to the partition wall is made curved, bubble formation at the lower end of the partition wall can be prevented with uniform introduction of the second fluid into the first sub-well.

The rapid cell culture test device of the present disclosure may be designed to be openable and closable through a fastening structure or may be provided with a cover film made of silicone, polyethylene terephthalate (PET), polyethylene (PE), polypropylene (PP), polystyrene (PS), polycarbonate (PC), Teflon or polyvinyl chloride (PVC). A cruciform or X-shaped punch may be formed at a position of the cover film corresponding to the underlying first sub-well 120. The punch can serve as an inlet portion through which a slight amount of the first fluid is easily introduced. A cruciform or X-shaped punch may be formed at a position of the cover film corresponding to the underlying second sub-well 130. The punch can serve as an inlet portion through which the second fluid is easily introduced. In terms of convenience of use, it is preferable that the shape of the inlet portion through which the first fluid is introduced is different from that of the inlet portion through which the second fluid is introduced.

Focus marks may be provided on the undersides of the first sub-wells to provide information on the positions of the target. The focus marks may be marked on the undersides of the first sub-wells such that the target areas of the cell culture test device can be automatically tracked. The focus marks are provided in the form of microchannels by injection molding. Since the biological agent is immobilized in solid thin films, the use of the focus marks enables imaging of the same areas every time pictures are taken. The focus marks serve to correct the positions of the target in the three axial directions, *i.e.* x-, y-, and z-axis directions, in the automatic tracking of target areas.

Hereinafter, a cell analysis method of the present disclosure will be described in detail.

According to a further aspect of the present disclosure, there is provided a cell analysis method using a cell culture test device including a plurality of well units, each of which includes an inlet portion through which a first fluid and a second fluid are introduced and a bottom portion including a first sub-well including a first fluid-accommodating space and a second sub-well in which the second fluid is accommodated and an antibiotic is loaded, wherein the first fluid-accommodating space has an edge groove formed along the outer circumference of the lower end thereof to accommodate a portion of the first fluid or increases in diameter in a direction from the upper end to the lower end thereof, the method including (a) introducing the first fluid into the first sub-well and gelling the mixture solution to form a solid thin film, (b) introducing the second fluid into the second sub-well to disperse or dissolve the antibiotic until the second fluid reaches the well unit over the second sub-well to come into contact with the solid thin film of the first fluid in the first sub-well such that the antibiotic is diffused into the solid thin film, and (c) observing changes of the biological agent on a single cell basis at the interface between the solid thin film of the first fluid and the second fluid.

First, a liquid medium containing a gelling agent is mixed with a biological agent to prepare a mixture solution as a first fluid.

The liquid medium may include at least about 95% by weight of water and can be gelled due to the presence of the gelling agent. The gelling agent may be, for example, agar, agarose, gelatin, alginate, collagen or fibrin. The use of agar or agarose is preferred. For example, agar may be used in an amount of 0.3 to 4% by weight in the liquid medium. The liquid medium usually requires no nutrients. In some examples, however, the liquid medium may include nutrients. The liquid medium may be a solution including a medium stimulating the division of particular cells.

Examples of suitable biological agents include viruses, bacteria, fungi, algae, protozoa, parasitic pathogens, human and mammalian cells, and biofilms. The biological agent may grow in a liquid or solid medium and the growth thereof may be affected by the kind and concentration of a foreign bioactive agent. The density of the biological agent in the mixture solution is from $10^2$ to $10^{10}$ cells/ml, preferably from $10^4$ to $10^9$ cells/ml, more preferably from $10^5$ to $10^8$ cells/ml. If the density of the biological agent is below the lower limit, it may be difficult to perceive the location of the biological agent. Meanwhile, if the density of the biological agent exceeds the upper limit, it may be difficult to perceive the individual state of the biological agent.

Thereafter, the first fluid is supplied to each of the first sub-wells. The supplied first fluid is introduced into the edge groove of the first fluid-accommodating space to prevent dislocation of the solid thin film, as described above. Instead of having the edge groove, the first fluid-accommodating space is designed to have a wall surface whose diameter increases in a direction from the upper end to the lower end thereof to prevent dislocation of the solid thin film. The first fluid can be supplied in an amount of 1 $\mu$l to 50 $\mu$l. After completion of the introduction, the first fluid is gelled. The resulting solid thin film immobilized with the biological agent. When the temperature of the liquid medium drops, the medium is gelled, which restricts the mobility of the biological agent. This immobilization facilitates continuous observation of the motile biological agent.

The thickness and width of the solid thin film are determined depending on the width of the first sub-well and the amount of the first fluid introduced. As used herein, the term "thin film" refers to a thin layer that has a thickness sufficient to immobilize the biological agent and to observe single cells. The thickness of the thin film is typically in the range of 1 $\mu$m to 5 mm, 1 $\mu$m to 3 mm, 1 $\mu$m to 2 mm, 1 $\mu$m to 1.5 mm, 1 $\mu$m to 1 mm, 1 $\mu$m to 800 $\mu$m, 1 $\mu$m to 500 $\mu$m, 1 $\mu$m to 100 $\mu$m, 10 $\mu$m to 3 mm, 100 $\mu$m to 500 $\mu$m, 10 $\mu$m to 1 mm, 100 $\mu$m to 1 mm, 200 $\mu$m to 1 mm or 500 $\mu$m to 1 mm, but is not particularly limited to this range. The thickness of the solid thin film may correspond to the size of a side of the solid thin film in a direction perpendicular to a side of the solid thin film to be observed. Within the thickness range of the solid thin film defined above, the biological agent immobilized in the solid thin film can be observed on a single cell basis.

Next, a solvent selected from water, cell culture media, dimethylformamide (DMF), and dimethyl sulfoxide (DMSO) is supplied as a second fluid to the second sub-well 134. The second fluid is supplied to disperse or dissolve an antibiotic loaded in the second sub-well 134. The second fluid fills up the bottom portion 130 and the lower portion 112. Thereafter, the second fluid is continuously supplied to fill the well unit.

After filling the second sub-well, the second fluid is supplied to the first sub-well and comes into contact with the solid thin film of the first fluid. As a result, the antibiotic dispersed or dissolved in the second fluid may diffuse into the solid thin film. As discussed above, due to the formation of the edge groove or the shape of the wall surface of the first fluid-accommodating space, the solid thin film can be prevented from being dislocated or displaced resulting from the introduction of the second fluid.

Next, responses of the biological agent to the antibiotic are observed. The biological agent immobilized in the solid thin film is distributed two-dimensionally, with the result that the biological agent can be observed on a single cell basis. According to the cell analysis method of the present disclosure, changes in the growth of the individual cells can be typically observed within several tens of minutes (normally 30 minutes). Accordingly, the cell analysis method of the present disclosure allows for the determination of the influence of the antibiotic on the biological agent in a more accurate and rapid manner than conventional methods. For example, the bioactivity of the antibiotic for bacterial cells can be tested within 3-4 hours. This rapid bioactivity test method is herein called single-cell morphological analysis (SCMA). The use of the cell culture test device enables observation of changes in single-cell morphology by time-lapse imaging under different antibiotic conditions.

An optical measurement system may be used for observation. The optical measurement system may include an imaging system, such as a CCD or CMOS camera. The optical measurement system may include optical units or devices necessary for focusing and light imaging, such as a lens, an illuminator, and a light guide. The optical measurement system may include an image processing system for processing and analyzing image data observed by the camera. The optical measurement system rapidly records and analyzes changes in the growth of the biological agent observed during testing to obtain test results. The area around the interface between the solid thin film of the first fluid and the second fluid is imaged. The imaging area may have a size of about 100 $\mu$m $\times$ 100 $\mu$m to 4000 $\mu$m $\times$ 4000 $\mu$m. It is preferable that the cross-sectional area of the first sub-well is at least larger in width than the imaging area.

Consequently, the use of the testing method based on the immobilization of the biological agent and the diffusion of the antibiotic can greatly reduce the amount of the drug and cells necessary for drug testing and enables rapid tracking of changes in the growth of single cells to obtain test results on the drug as rapidly as 2 hours (normally within 3-4 hours), compared to the prior art. This is the most rapid testing speed known thus far.

In one embodiment, the method may further include (d) observing changes of the biological agent responding to the antibiotic on a single cell basis to determine the minimum inhibitory concentration (MIC) or minimum biofilm eradication concentration (MBEC) of the antibiotic.

Biofilms are found in areas infected with microbes or to which microbes are attached. Biofilms refer to films that constitute mucilaginous microbial complexes, which are formed by microbes surrounded by polymer matrices. The formation of biofilms can greatly affect human health. Biofilms cause pulmonary infections, otitis media, periodontitis, and other infectious diseases. The resistance of bacteria present in biofilms to antibiotics is at least 1,000 times stronger than that of suspended bacteria. Flow cell systems and well-based systems have been used to investigate biofilms. However, these assay systems require a long time of several days for biofilm formation. Other difficulties associated with the use of the assay systems are that biofilms need to be stained and confocal microscopes should be used for observation. Further experiments are needed for the measurement of minimum inhibitory concentration (MIC) or minimum biofilm eradication concentration (MBEC). Such systems are very large in size and they fail to clearly show biofilm formation stages and to represent *in vivo* biofilm formation.

Thus, there is a need for efficient systems that are suitable to investigate the formation of biofilms and the reactivity of biofilms with antibiotics. In consideration of this need, the cell culture test device of the present disclosure proves to be an excellent alternative to conventional test devices.

Preferred embodiments of the present disclosure will be described in detail with reference to the accompanying drawings such that those skilled in the art can readily practice the disclosure. In describing the present disclosure, detailed explanations of a related known function or construction are omitted when it is deemed that they may unnecessarily obscure the essence of the disclosure. Certain features shown in the drawings are enlarged, reduced or simplified for ease of illustration and the drawings and the elements thereof are not necessarily in proper proportion. However, those skilled in the art will readily understand such details.

**Example 1**

A thermoplastic resin (polystyrene) was injection molded into the structure illustrated in FIGS. 1 and 2, which had an array of 12 $\times$ 8 aligned well units. A polypropylene film was attached to the rear surface of the structure to manufacture a chip for antibiotic susceptibility testing. The wall surfaces of the first fluid-accommodating spaces were made vertical. Each of the edge grooves was designed to have dimensions of 100 $\mu$m in thickness and 500 $\mu$m in width.

10 $\mu$l of a mixture of agarose and a bacterial solution as a first fluid was dispensed into the first fluid-accommodating space of the first sub-well of each of the well units. The first fluid was found to be normally introduced into the first fluid-accommodating space, the edge groove, and the sacrificial groove. After the introduction of the first fluid was completed, the first fluid was gelled. A culture medium as a second fluid was introduced into the second sub-wells in the same manner as described for the first fluid. The same amount (100 $\mu$l) of the second fluid was filled up to the stepped portion.

**Example 2**

The procedure of Example 1 was repeated except that the wall surface of each first fluid-accommodating space was designed to have an inverted slope at an angle of 30° with respect to the vertical plane.

### Example 3

The same procedure of Example 2 was repeated except that the edge grooves were omitted from the chip.

### Comparative Example 1

The procedure of Example 1 was repeated except that the wall surface of each first fluid-accommodating space was designed to have an inverted slope at an angle of 85° with respect to the vertical plane.

### Comparative Example 2

The procedure of Example 1 was repeated except that each of the edge grooves was designed to have dimensions of 0.5 $\mu$m in thickness and 200 $\mu$m in width.

### Comparative Example 3

The procedure of Example 1 was repeated except that each of the edge grooves was designed to have dimensions of 2500 $\mu$m in thickness and 2500 $\mu$m in width.

### Comparative Example 4

The same procedure of Example 1 was repeated except that the edge grooves were omitted from the chip.

### Test Example

In each of Examples 1-3 and Comparative Examples 1-4, 20 chips for antibiotic susceptibility testing having the same structure were manufactured (a total of 1920 wells). A determination was made as to whether the first and second fluids were successfully introduced. Introduction success or failure of the fluids was judged based on detachment of solid thin films, formation failure of solid thin films, and observation failure of the biological agent. The results are shown in Table 1.

[Table 1]

|  | Detachment of solid thin films | Formation failure of solid thin films | Observation failure |
|---|---|---|---|
| Example 1 | 0 | 3 | 0 |
| Example 2 | 0 | 12 | 0 |
| Example 3 | 0 | 13 | 0 |
| Comparative Example 1 | 0 | 508 | 1920 |
| Comparative Example 2 | 44 | 2 | 0 |
| Comparative Example 3 | 0 | 1920 | 0 |
| Comparative Example 4 | 82 | 4 | 0 |

As can be seen from the results in Table 1, the detachment of solid thin films was minimized, almost all solid thin films were formed without failure, and the biological agent was successfully observed in the chips of Examples 1 and 2. For the chips of Comparative Example 1 where the height of the first fluid-accommodating spaces was reduced, thin films of the first fluid were formed in the first sub-wells outside the first fluid-accommodating spaces, resulting in increased formation failure of solid thin films and observation failure of the biological agent. In the chips of Comparative Example 2 where the wall surfaces of the first fluid-accommodating spaces were made vertical and the edge grooves were reduced in size, solid thin films were detached from many wells. For the chips of Comparative Example 3 where the edge grooves were increased in size, the majority of the first fluid was introduced into the edge grooves and thin films were not formed in the first fluid-accommodating spaces, resulting in increased formation failure of solid thin films and observation failure of the biological agent. In the chips of Comparative Example 4 where the wall surfaces of the first fluid-accommodating spaces were made vertical and no edge grooves were formed, the largest number of thin films were detached. That is, the formation of edge grooves in the chips of Examples 1 and 2 was effective in preventing solid thin films from being detached. This effect was also maximized in the chips of Example 3 where the wall surfaces of the first fluid-accommodating spaces were inclined at a predetermined angle.

Although the particulars of the present disclosure have been described in detail, it will be obvious to those skilled in the art that such particulars are merely preferred embodiments and are not intended to limit the scope of the present disclosure. Therefore, the true scope of the present disclosure is defined by the appended claims and their equivalents.


**Claims**

1. A cell culture test device comprising a plurality of well units, each of which comprises an inlet portion through which a first fluid and a second fluid are introduced and a bottom portion comprising a first sub-well comprising a first fluid-accommodating space, wherein the first fluid-accommodating space has an edge groove formed along the outer circumference of the lower end thereof to accommodate a portion of the first fluid or increases in diameter in a direction from the upper end to the lower end thereof.

2. The cell culture test device according to claim 1, wherein the first fluid-accommodating space prevents the first fluid after gelation from being displaced or dislocated when the second fluid is supplied.

3. The cell culture test device according to claim 1, wherein the edge groove has a thickness of 1 to 2000 $\mu$m and a width of 300 to 2000 $\mu$m.

4. The cell culture test device according to claim 1, wherein the cell culture test device comprises bonding means disposed on the rear surface thereof to prevent leakage of the first fluid.

5. The cell culture test device according to claim 4, wherein the bonding means is an optically transparent film.

6. The cell culture test device according to claim 4, wherein the bonding means comprises a pressure control channel through which the first fluid is easily introduced into the first fluid-accommodating space and the edge groove.

7. The cell culture test device according to claim 1, wherein the bottom portion comprises a second sub-well in which the second fluid is accommodated and an antibiotic is loaded at a specific location.

8. The cell culture test device according to claim 1, wherein a stepped portion protrudes toward the center of the well unit along the circumference of the inner wall of the well unit such that the well unit has a lower portion whose inner width is smaller than that of an upper portion at the inlet portion side.

9. The cell culture test device according to claim 8, wherein the stepped portion has an angle of 90° or more with respect to the advancing direction of the second fluid such that the introduced second fluid is prevented from advancing vertically from the bottom portion.

10. The cell culture test device according to claim 8, wherein the stepped portion has a continuous edge shape along the circumference of the inner wall.

11. A cell analysis method using a cell culture test device comprising a plurality of well units, each of which comprises an inlet portion through which a first fluid and a second fluid are introduced and a bottom portion comprising a first sub-well comprising a first fluid-accommodating space and a second sub-well in which the second fluid is accommodated and an antibiotic is loaded, wherein the first fluid-accommodating space has an edge groove formed along the outer circumference of the lower end thereof to accommodate a portion of the first fluid or increases in diameter in a direction from the upper end to the lower end thereof, the method comprising (a) introducing the first fluid into the first sub-well and gelling the mixture solution to form a solid thin film, (b) introducing the second fluid into the second sub-well to disperse or dissolve the antibiotic until the second fluid reaches the well unit over the second sub-well to come into contact with the solid thin film of the first fluid in the first sub-well such that the antibiotic is diffused into the solid thin film, and (c) observing changes of the biological agent on a single cell basis at the interface between the solid thin film of the first fluid and the second fluid.

12. The cell analysis method according to claim 11, further comprising (d) observing changes of the biological agent responding to the antibiotic on a single cell basis to determine the minimum inhibitory concentration (MIC) or minimum biofilm eradication concentration (MBEC) of the antibiotic.

[FIG. 1]

(a)

(b) 130 134
132
142
140

(c)

[FIG. 2]

(a) 130 110 134 A'
132
A
142

(b) 110 120
140 114
142
112
132
130
150 135 160 134

[FIG. 3]

(a)

(b)

[FIG. 4]

[FIG. 5]

[FIG. 6]

[FIG. 7]

[FIG. 8]

[FIG. 9]

[FIG. 10]

[FIG. 11]

Radius of bottom face

[FIG. 12]

## INTERNATIONAL SEARCH REPORT

| International application No. |
| --- |
| **PCT/KR2020/013587** |

### A. CLASSIFICATION OF SUBJECT MATTER

**C12M 1/32**(2006.01)i; **C12M 1/12**(2006.01)i; **C12M 1/26**(2006.01)i; **C12M 1/34**(2006.01)i; **G01N 15/00**(2006.01)i

According to International Patent Classification (IPC) or to both national classification and IPC

### B. FIELDS SEARCHED

Minimum documentation searched (classification system followed by classification symbols)

C12M 1/32(2006.01); C12M 1/00(2006.01); C12M 3/00(2006.01); G01N 15/00(2006.01); G01N 33/50(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 세포배양(cell culture), 웰(well), 서브웰(sub well), 직경(diameter)

### C. DOCUMENTS CONSIDERED TO BE RELEVANT

| Category* | Citation of document, with indication, where appropriate, of the relevant passages | Relevant to claim No. |
| --- | --- | --- |
| Y | JP 2016-129495 A (DAINIPPON PRINTING CO., LTD.) 21 July 2016 (2016-07-21)<br>See paragraphs [0070] and [0072]; claim 1; and figure 5. | 1,2,4,5,7-12 |
| A | | 3,6 |
| Y | KR 10-1711105 B1 (QUANTAMATRIX INC.) 06 March 2017 (2017-03-06)<br>See paragraph [0048]; and claims 1, 15 and 18. | 1,2,4,5,7-12 |
| A | JP 3191179 U (SEIKOH GIKEN CO., LTD. et al.) 12 June 2014 (2014-06-12)<br>See entire document. | 1-12 |
| A | JP 2011-167101 A (STEM BIOMETHOD CORP.) 01 September 2011 (2011-09-01)<br>See entire document. | 1-12 |
| PX | KR 10-2127765 B1 (QUANTAMATRIX INC.) 29 June 2020 (2020-06-29)<br>See entire document. | 1-12 |

☐ Further documents are listed in the continuation of Box C.  ☑ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **13 January 2021** | **15 January 2021** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**INTERNATIONAL SEARCH REPORT**
Information on patent family members

International application No.

**PCT/KR2020/013587**

| Patent document cited in search report | | | Publication date (day/month/year) | Patent family member(s) | | | Publication date (day/month/year) |
|---|---|---|---|---|---|---|---|
| JP | 2016-129495 | A | 21 July 2016 | JP | 6511817 | B2 | 15 May 2019 |
| KR | 10-1711105 | B1 | 06 March 2017 | EP | 3434370 | A1 | 30 January 2019 |
| | | | | EP | 3434370 | A4 | 30 October 2019 |
| | | | | US | 2019-0100786 | A1 | 04 April 2019 |
| | | | | WO | 2017-183875 | A1 | 26 October 2017 |
| JP | 3191179 | U | 12 June 2014 | None | | | |
| JP | 2011-167101 | A | 01 September 2011 | None | | | |
| KR | 10-2127765 | B1 | 29 June 2020 | None | | | |

Form PCT/ISA/210 (patent family annex) (July 2019)